# EUROPÄISCHE PATENTSCHRIFT

(11) **EP 1 072 227 B1**
(45) Veröffentlichungstag und Bekanntmachung des Hinweises auf die Patenterteilung: **24.09.2003**
(21) Anmeldenummer: 00112473.4
(22) Anmeldetag: 10.06.2000
(51) Int. Cl.: A61B 17/68

(54) **Chirurgische Vorrichtung zum Verlagern von Knochenteilen**
Surgical device for displacement of bone parts
Dispositif chirurgical pour le déplacement de parties d'os

(30) Priorität: 24.07.1999 DE 19934889
(43) Veröffentlichungstag der Anmeldung: 31.01.2001
(73) Patentinhaber: Essiger, Holger K. Dr., 30900 Wedemark (DE)
(72) Erfinder: Essiger, Holger K. Dr., 30900 Wedemark (DE)
(74) Vertreter: Leine, Sigurd, Dipl.-Ing.

(56) Entgegenhaltungen:
- DE-A- 2 437 752
- DE-A- 19 500 202
- US-A- 5 129 903
- US-A- 5 700 263

## Beschreibung

Die Erfindung betrifft eine chirurgische Vorrichtung zum Verlagern von Knochenteilen, vorzugsweise jedoch eine Vorrichtung zur Kallusexpansion.

Insbesondere ist die Erfindung für die Kieferchirurgie bestimmt, und zwar zur Anwendung bei Athrophien bzw. Einziehungen und Schwunderscheinungen, bei denen es erforderlich ist, zum Zwecke der Regeneration Knochenteile abzutrennen und im Vergleich zum benachbarten Knochen oder insgesamt anzuheben bzw. den Abstand des Knochenteils zum Knochen zu vergrößern.

Bei bekannten Vorrichtungen dieser Gattung erfolgt eine Verstellung des Knochenteils durch eine Gewindeverstellung, wobei allerdings Elemente der Vorrichtung in die Mundhöhle des Patienten in einem solchen Maße hineinragen, daß die Anwendung der bekannten Vorrichtung als sehr störend empfunden wird und an den Schleimhaut- bzw. Hautdurchtrittsstellen Infektionsgefahr besteht.

US 5,700,263 (Basis für den Obergriff des Anspruchs 1.) offenbart eine chirurgische Vorrichtung zum Verlagern von Knochenteilen, mit zumindest einem Verstellelement, einem flachen, implantierbaren Gehäuse, in dem die für die Betätigung des Verstellelementes erforderliche Verstelleinrichtungen angeordnet sind, welche über eine Gehäusedurchbrechung von außen bedienbar sind, wobei das Verstellelement einen längsverschiebbaren Bolzen darstellt. Nachteilig ist hierbei der relativ hohe Platzbedarf der Vorrichtung bzw. des Verstellelementes.

DE 195 00 202 A1 offenbart einen Distraktor, insbesondere zur kranio-fazialen Kallusdistraktion, der ein erstes Verankerungselement und ein mit dem ersten Verankerungselement über eine Schieneneinrichtung verbundenes zweites Verankerungselement aufweist, wobei die fest mit dem ersten Verankerungselement verbundene Schieneneinrichtung mindestens ein Verzahnungselement aufweist und das zweite Verankerungselement eine mit dem Verzahnungselement der Schieneneinrichtung zusammenwirkende Verschiebeeinrichtung aufweist. Auch hierbei ist der relativ hohe Platzbedarf nachteilig.

Der Erfindung liegt die Aufgabe zugrunde, diese Nachteile durch einen besonderen Aufbau der Vorrichtung zu vermeiden.

Zur Lösung dieser Aufgabe ist erfindungsgemäß ein implantierbares, flaches Gehäuse vorgesehen, in dem sich eine Verstelleinrichtung für zumindest ein Verstellelement bzw. für zwei gegenläufig bewegbare Verstellelemente befindet, die über eine Gehäusedurchbrechung von außen her bedienbar sind, wobei das bzw. die Verstellelemente längsverschiebbare Bolzen sind, die auf lösbarer an den Knochen befestigte Widerlager einwirken. Dabei werden die Bolzen feinfühlig durch einen verschiebbaren Konus bewegt, da die Verstelleinrichtung einen mit seiner(n) Schrägfläche(n) auf die(den) Bolzen einwirkenden, verschiebbaren Konus aufweist. Die Verfahrbarkeit des Konus wird vorteilhafterweise durch ein von außen bedienbares Zahnrad oder Zahnsegment bewirkt, das mit einem auf einer am Konus befestigten Zahnstange kämmt, wobei vorteilhafterweise die Zahnstange (13) quasi eine Verlängerung der Längsmittelachse des Konus (12) ist.

Das Gehäuse wird dabei vorzugsweise an dem Knochen oder dem zu verstehenden (abzuhebenden) Knochenteil befestigt. Dies kann durch Anschrauben erfolgen.

Vorzugsweise wird allerdings das Gehäuse mit dem eigentlichen Knochen verbunden.

Wichtig ist somit, daß die Verstelleinrichtung gekapselt in einem Gehäuse untergebracht ist, das aufgrund seiner flachen Formgebung implantierbar ist. Diese Anordnung der Vorrichtung unterhalb des Weichgewebes stellt sicher, daß die Störung des Patienten auf ein Minimum herabgesetzt ist. Die erfindungsgemäße Vorrichtung ist somit patientenfreundlich und daher bei bisher nicht erschlossenen Fällen anwendbar.

Weitere Einzelheiten der Erfindung werden anhand der Zeichnung erläutert, in der ein Ausführungsbeispiel der Erfindung dargestellt ist.

Es zeigen:

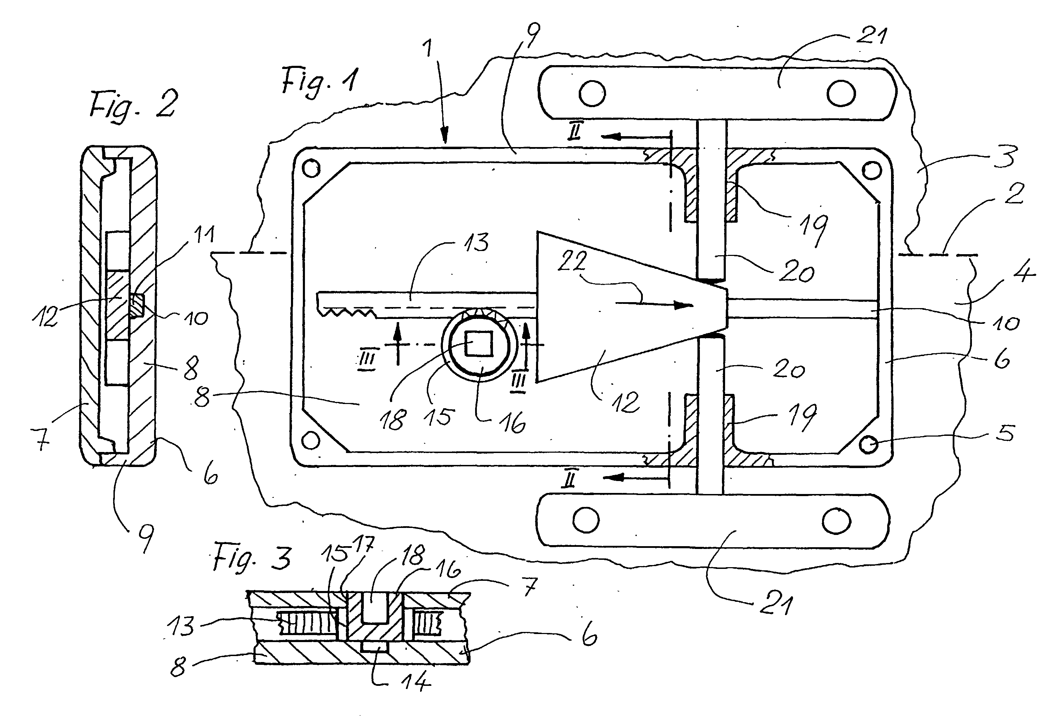

- Fig. 1: eine Vorrichtung zur Kallusexpansion im menschlichen Kieferbereich in Form eines flachen Gehäuses im Grundriß bei abgenommenem Deckel, und zwar im vergrößerten Maßstab,
- Fig. 2: einen Schnitt nach der Linie II - II von Fig. 1 und
- Fig. 3: einen Schnitt nach der Linie III - III von Fig. 1.

Ein wesentlicher Bestandteil der erfindungsgemäßen Vorrichtung ist ein im Grundriß flach rechteckiges Gehäuse 1 aus einem für die Implantation geeigneten Werkstoff z. B. Titan. Vorzugsweise sollen die Abmessungen etwa 3,5 - 5 cm X 1,5 - 2,5 cm bei einer Dicke von etwa 1,5 - 3 mm betragen, jedoch können ggf. diese Abmessungen für besondere Anwendungsfälle geändert werden. Dieses Gehäuse 1 soll unter dem Weichgewebe direkt auf dem Knochen aufliegen.

Die z. B. durch eine Säge herbeigeführte Knochentrennlinie ist mit 2 bezeichnet. Der abzuhebende Teil des Knochens hat das Bezugszeichen 3 und der eigentliche Knochen das Bezugszeichen 4. Im vorliegenden Falle soll das Gehäuse 1 auf dem Knochen 4 aufliegen. Zur Befestigung des Gehäuses 1 sind Löcher 5 vorgesehen, mit denen das Gehäuse 1 angeschraubt werden kann.

Das Gehäuse 1 besteht aus einem Unterteil 6 und einem Deckel 7, wobei diese Teile paßgenau ineinandergreifen und auch übliche Verschlußelemente vorgesehen sein können, die jedoch nicht dargestellt sind. Der Boden des Unterteils 6 ist mit 8 und die umlaufenden Seitenwände des Unterteils 6 sind mit 9 bezeichnet.

Der Boden 8 ist mit einer mittigen, längs zum Gehäuse 1 verlaufenden Nut 10 versehen, in die eine Rippe 11 eines symmetrischen Konus 12 eingreift, der in Verlängerung seiner Längsmittelachse eine sich nach hinten erstreckende Zahnstange 13 aufweist, mit der ein am Boden 8 mittels Zapfen 14 gelagertes Zahnrad 15 kämmt. Dieses Zahnrad erweitert sich nach oben hin über eine Nabe 16, die in einer runden Ausnehmung 17 des Deckels 7 gelagert ist. Die Nabe 16 ist zudem am freien Ende mit einem Vierkantloch 18 ausgespart, das natürlich auch durch das oft benutzte Sechskantloch ersetzt werden kann.

Dieses Vierkantloch 18 ist somit bei geschlossenem Gehäuse 1 von außen zugänglich. Dort kann ein geeigneter Schlüssel angesetzt werden, um das Zahnrad 15 zu verdrehen und damit den Konus 12 zu verschieben.

Zu beiden Seiten des Konus 12 sind in den Seitenwänden 9 Lagerstellen 19 für längsverschiebbare Bolzen 20 vorgesehen, die außerhalb des Gehäuses auf Widerlager 21 einwirken, welche streifenförmig ausgeführt sind und mit den Knochen 3, 4 fest verschraubt sind.

Die innerhalb des Gehäuses 1 befindlichen Enden der Bolzen 20 liegen auf den seitlichen Schrägflächen des Konus 12 auf. Aus diesem Grunde führt eine Konusbewegung im Sinne des Pfeiles 22 zu einer Verschiebung der Bolzen 20 nach außen und damit zu einer Anhebung des abzuhebenden Knochens 3.

Um Bewegungen des Konus 12 gegen die Richtung des Pfeiles 22 zu vermeiden, können Rücklaufsperren beliebiger Bauart vorgesehen sein, die auch von außen her lösbar sein müssen. Auf diese Sperren kann jedoch verzichtet werden, wenn aufgrund der Konussteigung Selbsthemmung gegeben ist.

Die Bolzen 20 können als lose Elemente gegeben sein. Sie können aber auch Bestandteil der Widerlager 21 sein. Wichtig ist allerdings, daß bei zwei Bolzen 20 eine gegenläufige Bewegung zum Verlagern der Knochen bzw. Ihrer Teile führt.

Die Verstelleinrichtung für die gegenläufigen Bolzen 20 sind demgemäß hermetisch verschlossen in dem Gehäuse 1 untergebracht. Darüber hinaus kann die Verstelleinrichtung aber ohne weiteres durch Verdrehen des Zahnrades 15 von außen her betätigt werden.

Wichtig ist ferner, daß das Gehäuse 1 sehr flach gehalten ist. Daher sind die hierin untergebrachten, bewegbaren Elemente 12, 13, 15 gegen einen unerwünschten Versatz gesichert. Sie finden ihren Anschlag an den Innenflächen des Gehäuses 1. Für die Bolzen 20 ist darüber hinaus durch die Lagerstellen 19 eine sichere Lagerung und Führung gegeben.

Es sei erwähnt, daß anstelle des in der Zeichnung dargestellten symmetrischen Konus auch ein asymmetrischer Konus (mit unterschiedlichen Steigungen) benutzt werden kann. Es sei weiterhin erwähnt, daß bei entsprechender Verankerung oder Halterung des Gehäuses 1 lediglich ein wirksames Verstellelement bzw. ein Bolzen 20 vorgesehen sein kann. Demgemäß kann also im Hinblick auf die zeichnerische Darstellung ein Bolzen 20 nicht wirksam sein bzw. ganz entfallen. In diesem Falle wäre ein nur einseitig wirkender Konus 12 ausreichend. Schließlich sei bemerkt, daß bei verankerten Widerlagern 21 eine Gehäusebefestigung überflüssig sein kann.

## Patentansprüche

1. Chirurgische Vorrichtung zum Verlagern von Knochenteilen, vorzugsweise zur Durchführung der Kallusexpansion, mit zumindest einem Verstellelement bzw. zwei gegenläufig bewegbaren Verstellelementen, wobei ein flaches, implantierbares Gehäuse (1) vorgesehen ist, in dem die für die Betätigung der(des) Verstellelemente(s) (20) erforderliche Verstelleinrichtungen (12,13) angeordnet sind, welche über eine Gehäusedurchbrechung (17) von außen bedienbar sind, wobei die(das) Verstellelement(e) (ein) längsverschiebbare(r) Bolzen (20) sind(ist),
**dadurch gekennzeichnet,**
**daß** die Verstelleinrichtung einen mit seinen(r) Schrägfläche(n) auf die(den) Bolzen (20) einwirkenden, verschiebbaren Konus (12) aufweist.

2. Vorrichtung nach Anspruch 1, **dadurch gekennzeichnet, daß** sich die Lagerstelle(n) für die(das) Verstellelement(e) (20) in den Seitenwänden (9) des Gehäuses (1) befinden.

3. Vorrichtung nach Anspruch 1, **dadurch gekennzeichnet, daß** der Konus (12) eine Zahnstange (13) aufweist, mit der ein von außen verdrehbares Zahnrad (15) kämmt.

4. Vorrichtung nach Anspruch 3, **dadurch gekennzeichnet, daß** der Konus (12) in Verlängerung seiner Längsmittelachse eine Zahnstange (13) aufweist, mit der ein von außen verdrehbares Zahnrad (15) kämmt.

5. Vorrichtung nach einem der Ansprüche 3 bis 4, **dadurch gekennzeichnet, daß** ein nabenähnlicher Fortsatz (16) in einen entsprechend eng bemessenen Durchbruch (17) des Gehäuses (1) eingreift, wobei der Fortsatz zum Ansetzen eines Werkzeuges oder derglechen ausgebildet ist.

6. Vorrichtung nach Anspruch 5, **dadurch gekennzeichnet, daß** der Fortsatz (16) mit einer Vertiefung (Vierkantloch 18 oder dergleichen) zum Ansetzen eines Werkzeuges versehen ist.

7. Vorrichtung nach einem oder mehreren vorhergehenden Ansprüchen, **dadurch gekennzeichnet, daß** das Gehäuse (1) derart flach ausgeführt und sein Inneres derart eng ist, daß die bewegbaren Teile (12,13,15) im Gehäuse durch die benachbarten Gehäuseinnenflächen an einem Verlassen ihrer Wirkstellung gehindert sind.

8. Vorrichtung nach Anspruch 1, **dadurch gekennzeichnet, daß** das Gehäuse (1) mit Durchbrechungen (Löcher 5) zum Ansetzen von Schrauben zur Gehäusebefestigung auf dem Knochen (3,4) ausgestattet ist.

9. Vorrichtung nach Anspruch 1, **dadurch gekennzeichnet, daß** die(der) Bolzen (20) mit am Knochen (3,4) angeschraubten Widerlager(n) (21) in Wirkverbindung stehen.

10. Vorrichtung nach Anspruch 1 und 3, **dadurch gekennzeichnet, daß** die längeren Seitenkanten des Gehäuses (1) parallel zur Längsmittelachse des Konus (12) verlaufen.

11. Vorrichtung nach Anspruch 1, **dadurch gekennzeichnet, daß** das Gehäuse (1) mit einem entfernbaren Deckel (7) versehen ist.

12. Vorrichtung nach Anspruch 1, **dadurch gekennzeichnet, daß** die Breite des Gehäuses (1) etwa 1,5 - 2,5 cm, seine Länge etwa 3,5 - 5 cm und seine Dicke etwa 1,5 - 3 mm betragen.

13. Vorrichtung nach Anspruch 1, **dadurch gekennzeichnet, daß** der Konus (12) mit einer vorspringenden Rippe (11) in einer Nut (10) des Gehäuses (1) geführt ist.

14. Vorrichtung nach Anspruch 13, **dadurch gekennzeichnet, daß** der Konus (12) mit einer vorspringenden Rippe (11) in einer Nut (10) des Gehäusebodens (8) geführt ist.

## Claims

1. A surgical device for the displacement of bone parts, preferably for performing callus expansion, having at least one adjustment element or two adjustment elements movable in opposite directions, wherein a flat implantable housing (1) is provided, in which the adjustment devices (12, 13) required for the actuation of the adjustment element(s) (20) are disposed, which can be operated from outside via a housing opening (17), wherein the adjustment element(s) is(are) (a) longitudinally displaceable pin(s) (20),
**characterised in that** the adjustment device comprises a sliding cone (12) acting with its inclined faces(s) on the pin(s) (20).

2. A device according to Claim 1,
**characterised in that** the mounting points(s) for the adjustment element(s) (20) are situated in the side walls (9) of the housing (1).

3. A device according to Claim 1,
**characterised in that** the cone (12) comprises a rack (13) with which a toothed wheel (15) that can be twisted from outside meshes.

4. A device according to Claim 3,
**characterised in that** the cone (12) in the extension of its longitudinal centre axis comprises a rack (13) with which a toothed wheel (15) that can be twisted from outside meshes.

5. A device according to one of Claims 3 to 4,
**characterised in that** a prolongation (16) similar to a hub engages in an opening (17), having correspondingly narrow dimensions, in the housing (1), the prolongation being constructed for fitting a tool or the like.

6. A device according to Claim 5,
**characterised in that** the prolongation (16) is provided with a recess (square hole 18 or the like) for fitting a tool.

7. A device according to one or more of the preceding Claims,
**characterised in that** the housing (1) has a flat design and its interior is narrow so that the movable parts (12, 13, 15) in the housing are prevented from leaving their active position by the adjacent inner faces of the housing.

8. A device according to Claim 1,
**characterised in that** the housing (1) is equipped with openings (holes 5) for fitting screws for attaching the housing to the bone (3, 4).

9. A device according to Claim 1,
**characterised in that** the pins(s) (20) actively communicate with abutment(s) (21) screwed to the bone (3, 4).

10. A device according to Claim 1 and 3,
**characterised in that** the longer side edges of the housing (1) run parallel to the longitudinal centre axis of the cone (12).

11. A device according to Claim 1,
**characterised in that** the housing (1) is provided with a removable cover (7).

12. A device according to Claim 1,
**characterised in that** the width of the housing (1) is roughly 1.5 - 2.5 cm, its length is roughly 3.5 - 5 cm and its thickness is roughly 1.5 - 3 mm.

13. A device according to Claim 1,
**characterised in that** the cone (12) is guided with a protruding rib (11) in a groove (10) of the housing (1).

14. A device according to Claim 13,
**characterised in that** the cone (12) is guided with a protruding rib (11) in a groove (10) in the housing base (8).

## Revendications

1. Dispositif chirurgical pour déplacer des parties d'os, de préférence pour la réalisation de l'expansion du cal osseux, avec au moins un élément de réglage ou respectivement deux éléments de réglage déplaçables en sens contraire, un boîtier (1) plat, implantable étant prévu, dans lequel les dispositifs de réglage (12, 13) nécessaires pour l'actionnement du(des) élément(s) de réglage (20) sont mis en place, lesquels dispositifs peuvent être commandés depuis l'extérieur à travers une ouverture (17) du boîtier, le(les) élément(s) de réglage étant une (des) cheville(s) (20) déplaçable(s) longitudinalement,
**caractérisé en ce que**
le dispositif de réglage présente une clavette coulissante (12) agissant sur la(les) cheville(s) (20) par sa(ses) surface(s) inclinée(s).

2. Dispositif selon la revendication 1,
**caractérisé en ce que** le(les) emplacement(s) de palier pour l'(les) élément(s) de réglage se trouvent dans les parois latérales (9) du boîtier (1).

3. Dispositif selon la revendication 1,
**caractérisé en ce que** la clavette (12) présente une crémaillère (13) sur laquelle engrène un pignon (15) entraînable en rotation depuis l'extérieur.

4. Dispositif selon la revendication 3,
**caractérisé en ce que** la clavette (12) présente dans le prolongement de son axe médian une crémaillère (13) sur laquelle engrène un pignon (15) entraînable en rotation depuis l'extérieur.

5. Dispositif selon l'une des revendications 3 à 4,
**caractérisé en ce qu'**un appendice similaire à un moyeu (16) est en prise dans un orifice (17) du boîtier (1) avec une dimension étroite correspondante, l'appendice étant formé pour recevoir un outil ou similaire.

6. Dispositif selon la revendication 5,
**caractérisé en ce que** l'appendice (16) est pourvu d'une cavité (trou carré 18 ou similaire) pour recevoir un outil.

7. Dispositif selon l'une ou plusieurs des revendications précédentes,
**caractérisé en ce que** le boîtier (1) est réalisé plat et son intérieur est étroit de sorte que les pièces mobiles (12, 13, 15) dans le boîtier soient empêchées par les surfaces internes adjacentes du boîtier de quiter leur position de fonctionnement.

8. Dispositif selon la revendication 1,
**caractérisé en ce que** le boîtier (1) est réalisé avec des ouvertures (trous 5) pour la mise en place de vis pour fixer le boîtier sur l'os (3, 4).

9. Dispositif selon la revendication 1,
**caractérisé en ce que** la(les) cheville(s) (20) se trouvent en liaison active avec un (des) contre-palier(s) (21) vissé(s) sur l'os (3, 4).

10. Dispositif selon la revendication 1 et 3, **caractérisé en ce que** les arêtes latérales les plus longues du boîtier (1) s'étendent parallèlement à l'axe médian longitudinal de la clavette (12).

11. Dispositif selon la revendication 1,
**caractérisé en ce que** le boîtier (1) est pourvu d'un couvercle amovible.

12. Dispositif selon la revendication 1,
**caractérisé en ce que** la largeur du boîtier (1) est d'environ 1,5 - 2, 5 cm, sa longueur d'environ 3,5 - 5 cm et son épaisseur d'environ 1,5-3 mm.

13. Dispositif selon la revendication 1,
**caractérisé en ce que** la clavette (12) est guidée par une nervure proéminente (11) dans une rainure (10) du boîtier (1).

14. Dispositif selon la revendication 13,
**caractérisé en ce que** la clavette (12) est guidée par une nervure proéminente (11) dans une rainure (10) du fond du boîtier (8).
